# EUROPEAN PATENT APPLICATION

(11) **EP 1 288 658 A2**
(43) Date of publication of application: **05.03.2003**
(21) Application number: 02256071.8
(22) Date of filing: 02.09.2002
(51) Int. Cl.: G01N 33/00, G01N 27/12, G01N 27/407

(54) **Gas sensor with proton conduction layer**

(30) Priority: 03.09.2001 JP 2001265755
(71) Applicant: NGK Spark Plug Company Limited, Nagoya, Aichi (JP)
(72) Inventor: Nadanami, Norihiko, Mizuho-ku, Nagoya, Aichi (JP); Kitanoya, Shoji, Mizuho-ku, Nagoya, Aichi (JP); Kondo, Tomonori, Mizuho-ku, Nagoya, Aichi (JP); Watanabe, Masaya, Mizuho-ku, Nagoya, Aichi (JP); Ishida, Noboru, Mizuho-ku, Nagoya, Aichi (JP)
(74) Representative: Nicholls, Michael John

(57) **Abstract**

A hydrogen gas sensor is configured such that a first electrode 3 is provided on one surface of a proton conduction layer 1; a second electrode 5 is provided on the other surface of the proton conduction layer 1 in opposition to the first electrode 3; and these components are supported in a support element. A conductive, elastic element 23 is disposed between a first lead portion 10a and a first electrode 3 in contact with the first lead portion 10a and the first electrode 3. The conductive, elastic element 23 is an electrically conductive, elastic sheetlike element made of metal, and has a pair of right-hand and left-hand through-holes 25 formed therein at a central portion thereof. The conductive, elastic element 23 is held between the first lead portion 10a and the first electrode 3 while being pressed inward by a first support element 9a. Thus, the first electrode 3 comes in close contact with the proton conduction layer 1, whereby electrical connection is established therebetween.

## Description

The present invention relates to a gas sensor such as a hydrogen sensor for measuring the concentration of hydrogen gas in a fuel gas for use in a fuel cell.

In response to concerns about global environmental pollution, in recent years intensive studies have been conducted on fuel cells for use as high-efficiency, clean power sources. Among such fuel cells, a polymer electrolyte fuel cell (PEFC) shows promise for automobile use and household use, by virtue of its inherent advantages, such as operation at low temperature and high output density.

A promising fuel gas for use in PEFC is a reformed gas. In this connection, in order to enhance efficiency and the like factor, a sensor (hydrogen gas sensor) capable of directly detecting hydrogen in a reformed gas must be provided. Since this hydrogen gas sensor is used in a hydrogen rich atmosphere, an operating temperature thereof must be low (about 100°C or lower).

Such a sensor of low-temperature operation type is proposed in, for example, European Patent No. 1103807A2. As shown in FIG. 5, the proposed sensor employs a proton conduction layer P1 formed from a polymer electrolyte (e.g., fluorine-containing resin) and is configured such that a first electrode P2 and a second electrode P3 are disposed on the corresponding surfaces of the proton conduction layer P1. The first electrode P2 and the second electrode P3 are elastic, porous electrodes which are formed from carbon that carries platinum or the like.

However, the proposed hydrogen gas sensor has sometimes involved the problem of low durability stemming from its configuration such that the first electrode P2, the second electrode P3, and the proton conduction layer P1, which are elastic, are held between paired support elements P4 and P5, and a lead portion P8 provided on the bottom surface of a recess P6 of the support element P4 is electrically connected to the first electrode P2 while a lead portion P9 provided on the bottom surface of a recess P7 of the support element P5 is electrically connected to the second electrode P3.

Specifically, in the course of use over a long period of time (long-term use), the elasticity of the first electrode P2 and that of the second electrode P3 are impaired, and thus a supporting effect exerted by the support elements P4 and P5 is weakened. As a result, the first electrode P2 or the second electrode P3 may separate from the proton conduction layer P1, or impaired conduction of electricity may arise between the first electrode P2 and the lead portion P8 or between the second electrode P3 and the lead portion P9.

Such impaired conduction of electricity or a like problem causes an increase in resistance between the electrodes P2 and P3, thus involving a difficulty in accurately measuring hydrogen gas concentration when the sensor is used over a long period of time.

An object of the invention is to provide a gas sensor capable of accurately measuring gas concentration such as hydrogen gas concentration over a long period of time and to solve the above-mentioned problems.

The present invention provides a gas sensor, comprising a support element adapted to support a first electrode and a second electrode, the first and second electrodes being provided in contact with a proton conduction layer, the support element comprising a lead portion electrically connected to a first electrode, a lead portion electrically connected to a second electrode, and a diffusion controlling portion for establishing communication between an atmosphere containing a gas to be measured and the first electrode (for controlling diffusion of gas). In this gas sensor, an object gas component (e.g., hydrogen gas) contained in the gas to be measured which is introduced from the atmosphere via the diffusion controlling portion is caused to be dissociated, decomposed, or reacted through application of voltage (sufficiently high for generating limiting current) between the first electrode and the second electrode to thereby generate protons, and concentration of the object gas component is obtained on the basis of limiting current generated as a result of the generated protons being pumped out via the proton conduction layer from the first electrode to the second electrode.

Particularly, the present invention is characterized in that a conductive, elastic element is disposed between at least either the first electrode or the second electrode and the lead portion (provided on the support element) corresponding to the electrode, while establishing electrical connection between the electrode and the lead portion corresponding to the electrode.

Specifically, in the present invention, a conductive, elastic element is disposed between at least either the first electrode or the second electrode; for example, the first electrode, the second electrode, or both the first and second electrodes, and the corresponding lead portion(s). Therefore, even when the elasticity of the first and second electrodes is impaired in the course of use over a long period of time, the conductive, elastic element maintains elasticity required for maintaining electrical connection, thereby preventing, for example, occurrence of insufficient contact between the first electrode or the second electrode and the corresponding lead portion when the sensor is used over a long period of time.

Therefore, an increase in resistance between the electrodes P2 and P3 can be prevented, whereby the concentration of an object gas component such as hydrogen gas can be accurately measured over a long period of time.

Preferably a reference electrode is provided in contact with the proton conduction layer and in opposition to the first electrode; and concentration of the object gas component is obtained on the basis of the limiting current in a state in which a voltage is applied between the first electrode and the second electrode such that electric potential between the first electrode and the reference electrode becomes constant.

In the present invention, the object gas component is caused to be dissociated, decomposed, or reacted through application of voltage (sufficiently high for generating limiting current) between the first electrode and the second electrode such that a potential difference between the first electrode and the reference electrode becomes constant, to thereby generate protons; and concentration of the object gas component is obtained on the basis of limiting current generated as a result of the generated protons being pumped out via the proton conduction layer from the first electrode to the second electrode.

The gas sensor of the present invention can prevent an increase in resistance between the first and second electrodes, whereby gas concentration can be accurately measured over a long period of time. Additionally, employment of the reference electrode enhances accuracy in measuring gas concentration.

The conductive, elastic element may comprise a gas passage portion for allowing passage of a gas to be measured (thus an object gas component).

Since the conductive, elastic element has a gas passage portion for allowing passage of the gas to be measured, even when the element is disposed between the lead portion and the electrode, the object gas component which is introduced via the diffusion controlling portion can readily reach the electrode.

Notably, since the diffusion controlling portion is usually responsible for controlling diffusion of gas, the gas passage portion does not assume a function for controlling diffusion of gas.

The gas passage portion can assume the form of a hole or a slit, or is formed of a porous material.

Preferably the gas sensor is a hydrogen gas sensor for measuring hydrogen gas concentration.

Also, the gas sensor may be used for measuring the concentration of hydrogen gas in a fuel gas for use in a polymer electrolyte fuel cell.

The present invention enables accurate measurement of the concentration of hydrogen gas in a fuel gas for use in a polymer electrolyte fuel cell without involvement of influence of, for example, methanol.

Notably, the gas sensor may be configured such that the proton conduction layer, the first electrode, the second electrode, and the diffusion controlling portion (as well as the reference electrode) are supported by the support element.

The diffusion controlling portion is adapted to control diffusion of a gas to be measured (particularly an object gas component) which is introduced into the gas sensor from an atmosphere containing the gas to be measured via the same, and may be implemented by, for example, a hole formed in the support element or a porous substance which fills the hole.

Fig. 1 Explanatory cutaway view showing a hydrogen gas sensor of Embodiment 1 of the present invention.

FIG. 2 Perspective views showing various examples of conductive, elastic elements for use in the hydrogen gas sensor of Embodiment 1 and the like gas sensor.

FIG. 3 Graph showing experiment results.

FIG. 4 Explanatory cutaway view showing a hydrogen gas sensor of Embodiment 2.

FIG. 5 Explanatory cutaway view showing a conventional hydrogen gas sensor.

### Description of Reference Numerals

- 1, 31: proton conduction layers
- 3, 33: first electrodes
- 5, 35: second electrodes
- 10a, 40a: first lead portions
- 10b, 40b: second lead portions
- 19, 49: diffusion controlling portions
- 23, 53, 63, 65, 71, 75: conductive, elastic elements
- 37: reference electrode
- 40c: third lead portion

### Examples of a mode for carrying out the present invention will next be described.

### Embodiment 1

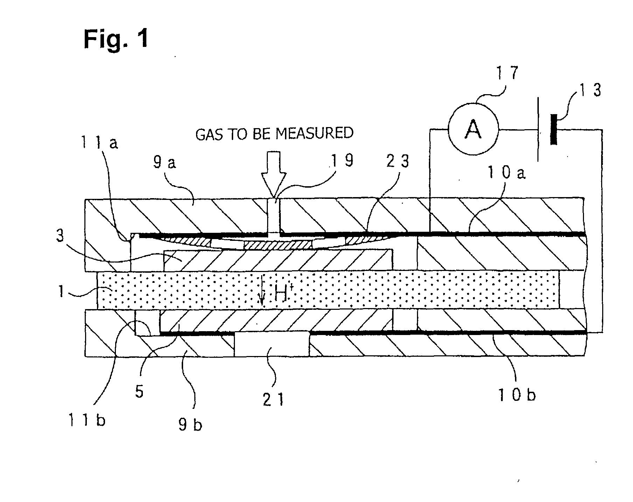

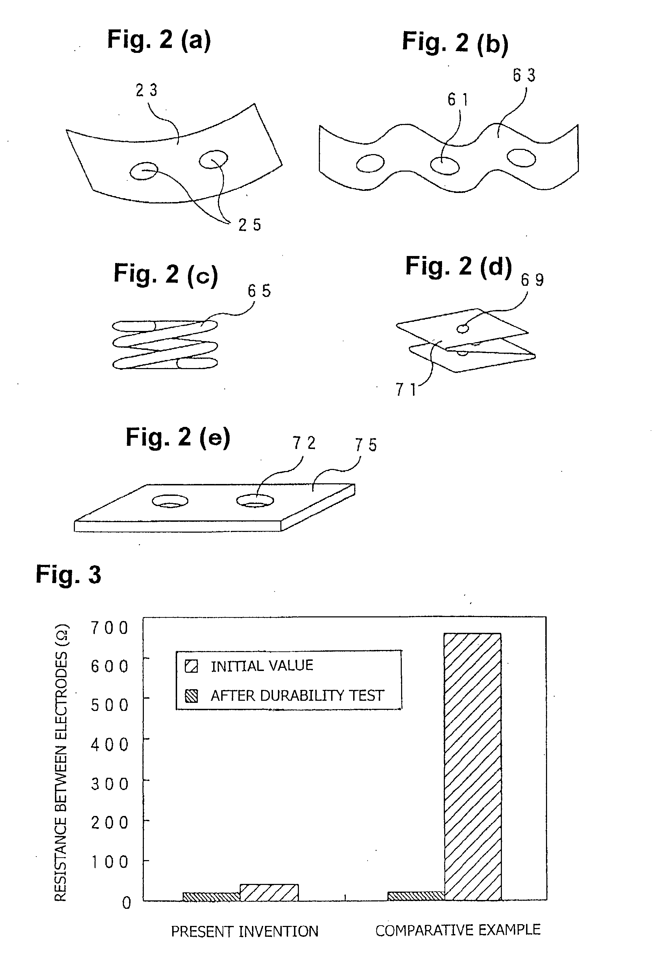

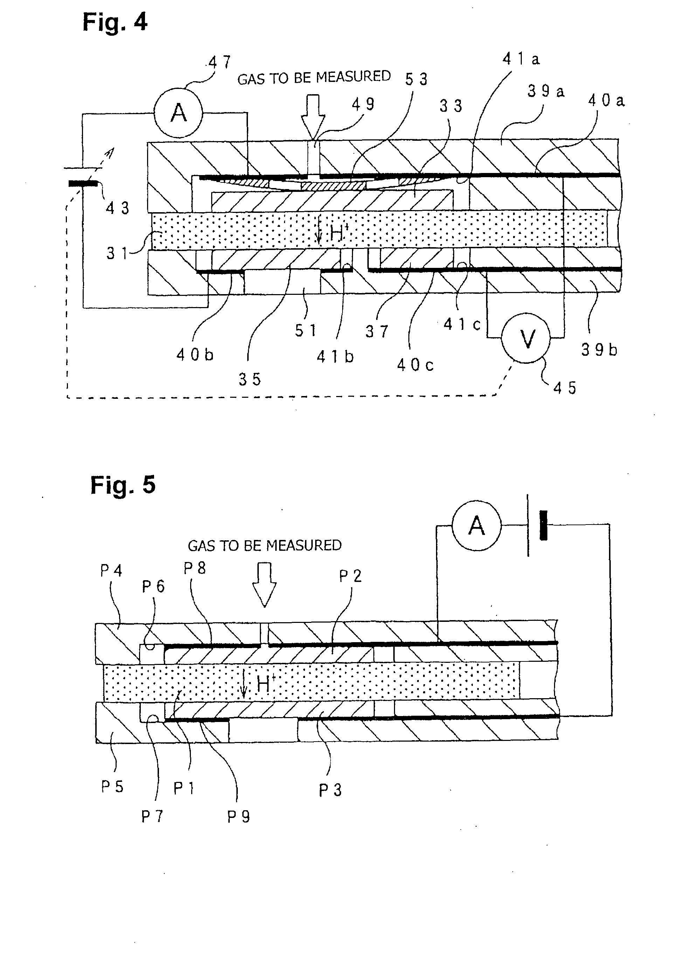

The present embodiment of a gas sensor is a hydrogen gas sensor used for measuring the concentration of hydrogen gas in a fuel gas for use in a polymer electrolyte fuel cell.
a) First, the configuration of the hydrogen gas sensor of the present embodiment will be described with reference to FIG. 1. FIG. 1 is a sectional view of the hydrogen gas sensor taken along the longitudinal direction thereof.
   As shown in FIG. 1, the hydrogen gas sensor of the present embodiment is configured such that a first electrode 3 is provided on one surface (upper surface in FIG. 1) of a proton conduction layer 1; a second electrode 5 is provided on the other surface (lower surface in FIG. 1) of the proton conduction layer 1 in opposition to the first electrode 3; and these components are supported in a support element consisting of a first support element 9a and a second support element 9b.
   Specifically, the proton conduction layer 1 is held between the first support element 9a and the second support element 9b; the first electrode 3 is covered by the first support element 9a while being disposed within a first recess 11a; and the second electrode 5 is covered by the second support element 9b while being disposed within a second recess 11b.
   The hydrogen gas sensor can be formed into a unitary body as follows. While the proton conduction layer 1 is held between the first support element 9a and the second support element 9b, the resultant assembly is fixed by means of an unillustrated fixing member or the like, or by means of a resin or the like.
   The proton conduction layer 1 is formed from a polymer electrolyte and can move protons (H⁺) through pumping from one side thereof to the other side thereof; for example, from the side toward the first electrode 3 to the side toward the second electrode 5. Preferably, the proton conduction layer 1 is formed of a material which allows operation at relatively low temperature (e.g., 150°C or lower). An example of such a material is Nafion (trade name, product of DuPont), which is a fluorine-containing resin.
   The first electrode 3 and the second electrode 5 are, for example, elastic, porous electrodes which contain a predominant amount of carbon. Each of the first electrode 3 and the second electrode 5 is coated with, for example, platinum on the side which comes into contact with the proton conduction layer 1. The platinum coating serves as a catalyst layer (not shown).
   The first electrode 3 and the second electrode 5 are connected to a circuit via a first lead portion 10a and a second lead portion 10b, respectively, such that a power supply (cell) 13 applies voltage between the first electrode 3 and the second electrode 5, and current which flows between the first electrode 3 and the second electrode 5 is measured by means of an ammeter 17.
   The support element is an insulator formed from, for example, ceramic which contains a predominant amount of alumina. In addition to an inorganic insulator formed from, for example, ceramic, an organic insulator formed from, for example, resin can be used as the support element .
   The first support element 9a, which partially constitutes the support element, has a diffusion controlling portion 19 for establishing communication between an ambient atmosphere and the first recess 1 1a (thus the first electrode 3). The diffusion controlling portion 19 is a small hole (e.g., diameter 0.06 mm) adapted to introduce to the side toward the first electrode 3 a fuel gas (thus hydrogen gas contained therein), which is a gas to be measured, and to control diffusion of the gas.
   The degree of controlling diffusion can be adjusted by adjusting the inside diameter of the diffusion controlling portion 19 or filling the diffusion controlling portion 19 with a porous material such as alumina.
   The second support element 9b has a hole 21 having a diameter of, for example, 1.7 mm formed therein for establishing communication between the ambient atmosphere and the second recess 11b (thus the second electrode 5).
   Each of the first support element 9a and the second support element 9b is formed by the steps of laminating sheets which contain ceramic and firing the resultant laminate. A layer formed from, for example, platinum is sandwiched between the sheets to form the lead portion 10a/10b in such a manner as to be exposed on the bottom of the recess 11a/11b.
   Particularly, in the present embodiment, a conductive, elastic element 23 is disposed between the first lead portion 10a (an exposed part thereof) and the first electrode 3 in contact with the first lead portion 10a and the first electrode 3. As shown in FIG. 2(a), the conductive, elastic element 23 is an electrically conductive, elastic sheetlike element made of metal, and has a pair of right-hand and left-hand through-holes 25 formed therein at a central portion thereof.
   As shown in FIG. 1, the conductive, elastic element 23 is held between the first lead portion 10a and the first electrode 3 while being pressed inward (downward in FIG. 1) by the first support element 9a. Thus, the first electrode 3 is pressed against the proton conduction layer 1 to come in close contact with the proton conduction layer 1, whereby electrical continuity is established along the lead portion 10a, the conductive, elastic element 23, the first electrode 3, and the proton conduction layer 1.
   Since the proton conduction layer 1 is thin and thus curves when pressed, the proton conduction layer 1 bends upon subjection to a pressing force induced by the elastic force of the conductive, elastic element 23, thereby establishing electrical connection not only between the proton conduction layer 1 and the second electrode 5 but also between the second electrode 5 and the second lead portion 10b.
b) Next will be described the principle of measurement and the procedure of measurement with respect to the hydrogen gas sensor of the present embodiment.
   When the hydrogen gas sensor is exposed to a fuel gas, hydrogen which has reached the first electrode 3 from an ambient atmosphere via the diffusion controlling portion 19 causes an electromotive force to be induced between the first electrode 3 and the second electrode 5 via the proton conduction layer 1 according to hydrogen gas concentration (specifically, according to a difference in hydrogen gas concentration between the side toward the first electrode 3 and the side toward the second electrode 5).
   The power supply 13 applies a voltage between the first electrode 3 and the second electrode 5.
   As a result, hydrogen is dissociated into protons on the first electrode 3; the thus-generated protons are pumped out to the second electrode 5 via the proton conduction layer 1 to become hydrogen again; and the thus-generated hydrogen diffuses into the atmosphere (outside the sensor).
   At this time, since current flowing between the first electrode 3 and the second electrode 5 (limiting current which is an upper limit current to be reached upon application of the aforementioned voltage) is proportional to hydrogen gas concentration, measuring the current enables determination of hydrogen gas concentration.
c) Next, a method for manufacturing the hydrogen gas sensor of the present embodiment will be briefly described.
   For example, as shown in FIG. 1, the second support element 9b is placed on a bench (not shown) with the second recess 11b thereof facing upward.
   Next, the proton conduction layer 1 with the first electrode 3 and the second electrode 5 being disposed on the corresponding opposite sides thereof is placed on the second support element 9b such that the second electrode 5 is accommodated in the second recess 11b.
   Next, the first support element 9a is disposed on the proton conduction layer 1 such that the first electrode 3 is enclosed by the first recess 11a.
   In this state; i.e., while the proton conduction layer 1 is held between the first support element 9a and the second support element 9b, the resultant assembly is press-fixed in the thickness direction thereof (in the vertical direction in FIG. 1) by means of an unillustrated fixing member or the like, thereby yielding a hydrogen gas sensor.
   The side faces of the hydrogen gas sensor are covered with, for example, a resin so as to seal the sensor except for the diffusion controlling portion 19, whereby introduction of gas is allowed only through the diffusion controlling portion 19. A sealing method is not limited thereto, so long as introduction of gas (to the side toward the first electrode 3) is allowed only through the diffusion controlling portion 19.
d) Next, the effect of the present embodiment will be described.
   As described above, the hydrogen gas sensor of the present embodiment is configured such that the conductive, elastic element 23 is disposed between the first lead portion 10a an the first electrode 3. Thus, even when the elasticity of the first and second electrodes 3 and 5, which are formed from carbon, is impaired in the course of long-term use, the conductive, elastic element 23 can press the first electrode 3 by imposing an appropriate elastic force on the same, thereby preventing separation of the first electrode 3 and the second electrode 5 from the proton conduction layer 1 and impaired conduction between the first electrode 3 and the lead portion 10a or between the second electrode 5 and the lead portion 10b.
   As a result, an increase in resistance between the first and second electrodes 3 and 5 can be suppressed, whereby hydrogen gas concentration can be accurately measured over a long period of time.
   Since the proton conduction layer 1 can bend when pressed, the mere disposition of the conductive, elastic element 23 between the first lead portion 10a and the first electrode 3 can reliably maintain electrical continuity over a long period of time not only along the first lead portion 10a, the first electrode 3, and the proton conduction layer 1 but also along the second lead portion 10b, the second electrode 5, and the proton conduction layer 1.
   Further, since two large through-holes 25 are formed in the conductive, elastic element 23 used in the present embodiment, even when the conductive, elastic element 23 is in contact under pressure with the lead portion 10a disposed in the first recess 11a, the conductive, elastic element 23 does not suppress or control diffusion of a gas to be measured which is introduced to the side toward the first electrode 3 via the diffusion controlling portion 19.
d) Next will be described an experiment which was carried out for confirming the effect of the present embodiment.
   This experiment was intended to study the influence of presence/absence of a conductive, elastic element on long-term durability.
   (1) First, a hydrogen gas sensor including a conductive, elastic element was manufactured in a manner similar to that of Embodiment 1, as an example of the present invention.
      As a Comparative Example which falls outside the scope of the present invention, a hydrogen gas sensor as shown in FIG. 5 was manufactured in a manner similar to that of Embodiment 1 except that a conductive, elastic element is not employed.
   (2) The hydrogen gas sensor of Embodiment 1 and that of Comparative Example were subjected to a 500-hour durability test, and examined for a change in resistance between electrodes in the course of the test.
      Specifically, in measurement of the concentration of hydrogen gas in a gas to be measured which had the gas composition specified below, by use of these hydrogen gas sensors, resistance between the first electrode and the second electrode was measured before and after the durability test. Measuring conditions are as follows.
      · Gas composition: H₂=50%, H₂O=20%, N₂=bal.
      · Gas temperature: 80°C
      · Gas flow rate: 10 L/min
      · Voltage applied between two electrodes: 50 mV
      · Durability test period of time: 500 hours
   (3) Measurement results are shown in FIG. 3.

As is apparent from FIG. 3, the hydrogen gas sensor of Embodiment 1, which falls within the scope of the present invention; i.e., the hydrogen gas sensor which employs the conductive, elastic element, exhibits a merely small increase in the resistance after the 500-hour durability test, as compared with the hydrogen gas sensor of Comparative Example, which does not employ the conductive, elastic element.

The above-described test has revealed that a hydrogen gas sensor equipped with a conductive, elastic element as in the case of Embodiment 1, which falls within the scope of the present invention, exhibits a merely small increase in resistance between the first electrode and the second electrode after a long-term durability test, indicating that the sensor can accurately measure hydrogen gas concentration over a long period of time.

### Embodiment 2

Embodiment 2 will next be described. However, repeated description of features similar to those of Embodiment 1 will be omitted.

The hydrogen gas sensor of the present embodiment assumes the configuration of Embodiment 1 to which a reference electrode is added.
a) First, the configuration of the hydrogen gas sensor of the present embodiment will be described with reference to FIG. 4. FIG. 4 is a sectional view of the hydrogen gas sensor taken along the longitudinal direction thereof.
   As shown in FIG. 4, the hydrogen gas sensor of the present embodiment is configured such that a first electrode 33 is provided on one surface (upper surface in FIG. 4) of a proton conduction layer 31; a second electrode 35 and a reference electrode 37 are provided on the other surface (lower surface in FIG. 4) of the proton conduction layer 31 in opposition to the first electrode 33; and these components are supported in a support element consisting of a first support element 39a and a second support element 39b.
   Specifically, the proton conduction layer 31 is held between the first support element 39a and the second support element 39b; the first electrode 33 is covered by the first support element 39a while being disposed within a first recess 41a; the second electrode 35 is covered by the second support element 39b while being disposed within a second recess 41b; and the reference electrode 37 is covered by the second support element 39b while being disposed within a third recess 41c.
   The proton conduction layer 31 is formed from a polymer electrolyte and can move protons (H⁺) through pumping from one side thereof to the other side thereof; for example, from the side toward the first electrode 33 to the side toward the second electrode 35.
   The first electrode 33, the second electrode 35, and the reference electrode 37 are, for example, porous electrodes which contain a predominant amount of carbon. Each of the electrodes 33, 35, and 37 is coated with, for example, platinum on the side which comes into contact with the proton conduction layer 31. The platinum coating serves as a catalyst layer (not shown).
   The first electrode 33, the second electrode 35, and the reference electrode 37 are connected to a circuit via a first lead portion 40a, a second lead portion 40b, and a third lead portion 40c, respectively, such that a power supply (cell) 43 applies a voltage between the first electrode 33 and the second electrode 35; the voltage applied between the first electrode 33 and the reference electrode 37 is measured by means of a voltmeter 45; and the current which flows between the first electrode 33 and the second electrode 35 is measured by means of an ammeter 47.
   The reference electrode 37 is used such that, by maintaining the voltage between the first electrode 33 and the reference electrode 37 at a constant level, there is reduced the influence of disturbances such as temperature and humidity on measurement of the concentration of hydrogen gas in a gas to be measured. Preferably, in order to stabilize hydrogen concentration on the reference electrode 37, the reference electrode 37 is a self-generation-type reference electrode.
   The support element is an insulator formed from, for example, ceramic which contains a predominant amount of alumina. The first support element 39a, which partially constitutes the support element 39, has a diffusion controlling portion 49 for establishing communication between an ambient atmosphere and the first recess 41a.
   The second support element 39b has a hole 51 for establishing communication between the ambient atmosphere and the second recess 41b.
   Each of the first support element 39a and the second support element 39b is formed by laminating sheets which contain ceramic. Each of the lead portions 40a to 40c is formed between the laminated sheets such that the lead portions 40a to 40c are exposed on the bottoms of the recesses 41a to 41c, respectively, so as to establish electrical connection to the corresponding electrodes 33, 35, and 37.
   Also, in the present embodiment, a conductive, elastic element 53 similar to that of Embodiment 1 is disposed between the first lead portion 40a and the first electrode 33 in contact with the first lead portion 40a and the first electrode 33, thereby establishing electrical connection between the first lead portion 40a and the first electrode 33.
b) Next will be described the principle of measurement and the procedure of measurement with respect to the hydrogen gas sensor of the present embodiment.
   When the hydrogen gas sensor is exposed to a fuel gas, hydrogen which has reached the first electrode 33 from an ambient atmosphere via the diffusion controlling portion 49 causes an electromotive force to be induced between the first electrode 33 and the reference electrode 37 via the proton conduction layer 31 according to hydrogen gas concentration (specifically, according to a difference in hydrogen gas concentration between the side toward the first electrode 33 and the side toward the reference electrode 37).
   The power supply 43 applies an appropriate voltage between the first electrode 33 and the second electrode 35 such that potential difference difference between the first electrode 33 and the reference electrode 37 becomes constant.
   Specifically, hydrogen gas concentration on the first electrode 33 is controlled at a constant level by varying the voltage applied between the first electrode 33 and the second electrode 35 to an optimum level with the concentration of hydrogen gas in a gas to be measured. For example, when the concentration of hydrogen gas in the gas to be measured is high, the voltage applied between the first electrode 33 and the second electrode 35 is increased; and when the hydrogen gas concentration is low, the voltage is decreased. Also, when variation of, for example, the temperature of a gas to be measured causes an increase in resistance between the first electrode 33 and the second electrode 35, the applied voltage is varied as appropriate so as to control the hydrogen gas concentration on the first electrode 33 at a constant level.
   As a result, hydrogen is dissociated into protons on the first electrode 33; the thus-generated protons are pumped out to the second electrode 35 via the proton conduction layer 31 to become hydrogen again; and the thus-generated hydrogen diffuses into the atmosphere.
   At this time, since current flowing between the first electrode 33 and the second electrode 35 (limiting current which is an upper limit current to be reached upon application of the aforementioned voltage) is proportional to hydrogen gas concentration, measuring the current enables determination of hydrogen gas concentration.
   Particularly, by setting the potential difference between the first electrode 33 and the reference electrode 37 to an optimum value, even in application to an atmosphere involving a great variation of, for example, temperature, hydrogen gas concentration on the first electrode 33 can be always adjusted to an optimum valve, whereby hydrogen gas concentration can be measured at higher accuracy (as compared with the case where the reference electrode 37 is not employed).
c) Next, the effect of the present embodiment will be described.
   As in the case of Embodiment 1, the hydrogen gas sensor of the present embodiment is configured such that the conductive, elastic element 53 is disposed between the first lead portion 40a an the first electrode 33, thereby avoiding occurrence of impaired conduction in the course of use over a long period of time and providing capability of accurately measuring hydrogen gas concentration over a long period of time.

Particularly, the present embodiment employs the reference electrode 37, and thus can measure hydrogen gas concentration at higher accuracy.

The present invention is not limited to the above-described embodiments, but may be embodied in many other specific forms without departing from the spirit or scope of the invention.
(1) For example, Embodiments 1 and 2 are described while mentioning a hydrogen gas sensor for measuring the concentration of hydrogen gas in a fuel gas. However, the gas sensor of the present invention can also be applied to the case of measuring the concentration of carbon monoxide or methanol gas in a fuel gas.
(2) Embodiments 1 and 2 are described while mentioning the conductive, elastic element disposed between the first electrode and the first support element. However, the conductive, elastic element may be disposed between the second electrode and the second support element. Alternatively, the conductive, elastic element may be disposed not only between the first electrode and the first support element but also between the second electrode and the second support element. Notably, the conductive, elastic element may be disposed between the reference electrode and the second support element.
(3) In place of the conductive, elastic element used in Embodiments 1 and 2, the conductive, elastic elements shown in FIGS. 2(b) to 2(e) may be used.

Specifically, a conductive, elastic element 63 shown in FIG. 2(b) may be employed. The conductive, elastic element 63 is a corrugated sheet made of metal or the like and has a plurality of through-holes 61 formed therein. A conductive, elastic element 65 shown in FIG. 2(c) may be employed. The conductive, elastic element 65 is a coil spring formed of, for example, a metallic wire. A conductive, elastic element 71 shown in FIG. 2(d) may be employed. The conductive, elastic element 71 is a spring formed by folding, for example, a metallic sheet in layers and has a through-hole 69 formed in each layer. A conductive, elastic element 75 shown in FIG. 2(e) may be employed. The conductive, elastic element 75 is a rubberlike elastic material having electrical conductivity and has through-holes 72 formed therein.

## Claims

1. A gas sensor, comprising:
a support element adapted to support a first electrode (3, 33) and a second electrode (5, 35), the first and second electrodes being provided in contact with a proton conduction layer (1, 31),
the support element comprising:
a first lead portion (10a, 40a) electrically connected to said first electrode,
a second lead portion (10b, 40b) electrically connected to said second electrode, and
a diffusion controlling portion (19, 49) for establishing communication between an atmosphere containing a gas to be measured and the first electrode
wherein an object gas component contained in the gas to be measured which is introduced from the atmosphere via the diffusion controlling portion is dissociatable, decomposable, or reactable through application of voltage between said first electrode and said second electrode to thereby generate protons, and
concentration of the object gas component is obtainable on the basis of a limiting current generated as a result of the generated protons being pumped out via the proton conduction layer from said first electrode to said second electrode, and
wherein a conductive, elastic element (23, 53, 63, 65, 71, 75) is disposed between at least either said first electrode or said second electrode and the respective first or second lead portion and establishing electrical connection between the electrode and the respective lead portion.

2. The gas sensor as described in claim 1, wherein a reference electrode (37) is provided in contact with the proton conduction layer and in opposition to the first electrode; and
wherein concentration of the object gas component is obtainable on the basis of the limiting current on application of a voltage between the first electrode and the second electrode such that a potential difference between the first electrode and the reference electrode becomes constant.

3. The gas sensor as described in claim 1 or 2, wherein the conductive, elastic element comprises a gas passage portion for allowing a concentration of a continuous passage of the gas to be measured.

4. The gas sensor as described in claim 3, wherein the gas passage portion assumes the form of a hole or a slit, or is formed of a porous material.

5. The gas sensor as described in claim 1, 2 or 3, wherein the gas sensor is a hydrogen gas sensor for measuring hydrogen gas concentration.

6. The gas sensor as described in claim 5, wherein the gas sensor is used for measuring the concentration of hydrogen gas in a fuel gas for use in a polymer electrolyte fuel cell.
